# EUROPEAN PATENT SPECIFICATION

(11) **EP 4 512 439 B1**
(45) Date of publication and mention of the grant of the patent: **15.07.2026**
(21) Application number: 24190780.7
(22) Date of filing: 25.07.2024
(51) Int. Cl.: A61L 27/36

(54) **DECELLULARIZED MATRIX MATERIAL AND PREPARATION METHOD THEREFOR AND USE THEREOF**
DEZELLULARISIERTES MATRIXMATERIAL UND HERSTELLUNGSVERFAHREN DAFÜR UND VERWENDUNG DAVON
MATÉRIAU DE MATRICE DÉCELLULARISÉE, SON PROCÉDÉ DE PRÉPARATION ET SON UTILISATION

(30) Priority: 26.07.2023 CN 202310924645
(43) Date of publication of application: 26.02.2025
(73) Proprietor: Jiangsu Brightness Medical Devices Co., Ltd., Changzhou Jiangsu 213013 (CN)
(72) Inventor: ZHANG, Jintong, Changzhou, 213013 (CN); ZHANG, Peng, Changzhou, 213013 (CN); LIU, Pengfei, Changzhou, 213013 (CN)
(74) Representative: Cabinet Didier Martin

(56) References cited:
- CN-B- 115 337 260
- US-B2- 7 550 152

## Description

### TECHNICAL FIELD

The present application belongs to the field of biotechnology and relates to a decellularized matrix material and a preparation method therefor and use thereof.

### BACKGROUND

With the further development of tissue engineering and the in-depth study of bio-patch endogenous regeneration mechanisms, bio-patch has begun to emerge in the tissue damage repair. The main structure of the bio-patch is a fibrous scaffold of extracellular matrix composed of collagen, elastic fibers, glycoproteins, laminins, and proteoglycans, and such structure provides an ideal scaffold for tissue regeneration.

When implanted into the body, the bio-patch will induce tissue to undergo endogenous tissue regeneration, which mainly includes the patch of rapid revascularization, and the stem cells in circulation entering the patch and differentiating, filling, and producing new extracellular matrix, thereby repairing the tissue defect. In the process, the porous structure of bio-patch facilitates the growth and attachment of the above-mentioned stem cells; meanwhile, the bio-patch can be completely absorbed, does not injure the around tissues or organs, and has a good ability to tolerate the infection without increasing the risk of postoperative infection, which are also advantages of the bio-patch over some conventional polymer materials. The bio-patch can be used as the anastomat reinforcement patch, hernia patch, dura mater patch, and the like.

Biomaterials need to undergo strict sterilization, inactivation, and decellularization treatment before being used as qualified patch products. At present, the decellularization techniques include physical repeated freezing and thawing, physical swelling, chemical decellularization, enzymatic digestion, etc. Simple physical repeated freezing and thawing and swelling are not only time-consuming and labor-intensive, but at the same time, they will cause great damage to the collagen structure; due to the high concentration of chemical regents, the chemical decellularization will damage the collagen structure, and also have residual reagents; the enzymatic decellularization has extremely low efficiency. For example, CN103272275A discloses a preparation method for a dura mater bio-patch, including separating small intestinal submucosa, virus inactivation, and decellularization; the decellularization is carried out in a thermostatic ultrasonic cleaner in which the cleaning tank can be oscillated, and the small intestinal submucosa is treated in a sodium hydroxide solution. Document CN-115 337 260 B discloses a composite decellularized scaffold/hyaluronic acid temperature-sensitive hydrogel and application thereof.

In summary, it's one of the urgent problems in the field of tissue engineering still needs to be solved how to develop new preparation methods for decellularized matrix materials to obtain highly qualified decellularized materials.

### SUMMARY

In view of the deficiencies of the prior art and practical needs, the present application provides a decellularized matrix material and a preparation method therefor and use thereof. The processed decellularized matrix material has good biocompatibility, large porosity and intact collagen structure of the tissue; and the preparation method effectively reduces exogenous sensitizing antigens and the process is relatively mild.

In a first aspect, the present application provides a preparation method for a decellularized matrix material, and the preparation method includes:
subjecting small intestinal submucosa to pre-treatment, and subjecting the small intestinal submucosa after the pre-treatment to swelling treatment, freeze-thaw treatment, oxidase immersion treatment, and surfactant treatment in sequence, so as to obtain the decellularized matrix material.

In the present application, the combination of physical swelling, physical freezing and thawing, and chemical elution guarantees a high decellularization efficiency; moreover, by adding the oxidase at an appropriate time during the decellularization treatment process, the oxidase thoroughly permeates into the decellularized matrix, and thereby plays a buffering role in the tissue when the surfactant is applied, ensuring the intactness of the collagen structure in the extracellular matrix.

It is to be understood that the underneath layer of small intestinal tissue of mammals such as pigs, cows, sheep, etc. are suitable for the present application, and the small intestinal tissue may be treated in a common manner in the art to obtain the underneath layer of small intestinal tissue.

Preferably, the pre-treatment includes washing and drying.

Preferably, the pre-treatment further includes bacteria and viruses inactivation.

Preferably, the bacteria and viruses inactivation includes: irradiating the small intestinal submucosa with ultraviolet light, then incubating with an antibiotic solution, washing, and incubating with peroxyacetic acid.

Preferably, the antibiotic solution contains any one or a combination of at least two of gentamicin, vancomycin, penicillin, streptomycin, cefoxitin, or chloramphenicol.

Preferably, the antibiotic solution has a total concentration of 1-100 mg/L, including 2 mg/L, 3 mg/L, 4 mg/L, 5 mg/L, 6 mg/L, 7 mg/L, 8 mg/L, 20 mg/L, 50 mg/L, 60 mg/L, 80 mg/L, 90 mg/L, 92 mg/L, 95 mg/L, 97 mg/L, or 99 mg/L.

The swelling treatment includes: mixing and incubating the small intestinal submucosa with a hypertonic solution.

The hypertonic solution includes any one or a combination of at least two of a ZnCl₂ solution, a NaCl solution, a CaCl₂ solution, a KCl solution, or a MgCl₂ solution.

Preferably, the incubation is performed for a period of 2-5 hours at a temperature of 20-40°C (such as 21°C, 22°C, 25°C, 26°C, 27°C, 28°C, 37°C, 38°C, or 39°C).

In the present application, the use of hypertonic ZnCl₂ solution for swelling can dilate the tissue to a larger volume, and thereby leading to a larger pore structure, increasing the porosity of the tissue; moreover, ZnCl₂ has a certain degree of oxidizing ability, and is capable of removing the fat or residual epidermal structure in the tissue, further improving the porosity and purification efficiency.

Preferably, the ZnCl₂ solution has a concentration of 3-6 M, including 4 M or 5 M.

Preferably, the freeze-thaw treatment includes: incubating the small intestinal submucosa at -80°C to -20°C (such as -79°C, -75°C, -70°C, -60°C, -50°C, -40°C, -35°C, -30°C, -25°C, -24°C, -23°C, -22°C, or -21°C) for 2-24 hours (such as 3 hours, 4 hours, 5 hours, 6 hours, 7 hours, 8 hours, 9 hours, 15 hours, 20 hours, 21 hours, 22 hours, or 23 hours), then allowing the small intestinal submucosa to thaw in normal saline at 20-30°C (such as 21°C, 22°C, 23°C, 25°C, 26°C, 27°C, 28°C, or 29°C), and repeating the freeze-thaw cycle for 3-5 times.

Preferably, the oxidase includes any one or a combination of at least two of lipoxygenase, urate oxidase, D-amino acid oxidase, L-amino acid oxidase, or L-α-hydroxy acid oxidase.

In the present application, the oxidase is used to penetrate into the matrix tissue and plays a buffering effect on the tissue, which is conducive to protecting the collagen structure of the matrix from being damaged.

Preferably, the oxidase has a working concentration of 0.05-10 mg/mL, including 0.06 mg/mL, 0.07 mg/mL, 0.08 mg/mL, 0.1 mg/mL, 0.5 mg/mL, 1 mg/mL, 2 mg/mL, 3 mg/mL, 4 mg/mL, 5 mg/mL, 6 mg/mL, 7 mg/mL, 8 mg/mL, or 9 mg/mL.

Preferably, the surfactant includes any one or a combination of at least two of a saponifiable matter, polyethylene glycol, or polysorbate (Tween).

Preferably, the polyethylene glycol includes PEG400 to PEG2000.

Preferably, the polysorbate includes Polysorbate 60 to Polysorbate 80.

Preferably, the surfactant has a working concentration of 0.05-5 mg/mL, including 0.06 mg/mL, 0.07 mg/mL, 0.08 mg/mL, 0.1 mg/mL, 0.5 mg/mL, 1 mg/mL, 1.5 mg/mL, 2 mg/mL, 2.5 mg/mL, 3 mg/mL, 4 mg/mL, 4.5 mg/mL, 4.8 mg/mL, or 4.9 mg/mL.

Preferably, ratios of the small intestinal submucosa to each of the treatment solutions (such as the normal saline, the antibiotic solution, the hypertonic solution, and the oxidase solution) are all within 1: (50-200) (v/v), including 1: 100, 1: 150, 1: 160, 1: 180, or 1: 190.

Preferably, after the surfactant treatment, the preparation method further includes steps of fixation molding and vacuum lyophilizing.

Preferably, the fixation molding includes: placing at least one layer of small intestinal submucosa on a mold.

Preferably, the vacuum lyophilizing includes: lyophilizing the mold containing the small intestinal submucosa in a lyophilizer.

Preferably, a procedure of the lyophilizing includes: pre-freezing to -45°C to -25°C, and holding for 1-2 hours; setting a pressure to 20-25 Pa, and adjusting a temperature to -25°C to -15°C and holding for 5-7 hours; and finally adjusting a temperature to 0°C, and holding for 4-6 hours.

As a preferred technical solution, the preparation method for a decellularized matrix material includes the following steps:
(1) washing and drying the small intestinal submucosa;
(2) irradiating the small intestinal submucosa after being treated in step (1) with ultraviolet light, then incubating with an antibiotic solution, washing, incubating with peroxyacetic acid, and washing and drying;
(3) mixing the small intestinal submucosa after being treated in step (2) with a 3-6 M ZnCl₂ solution and incubating at 20-30°C for 2-5 hours, and washing; subsequently incubating the small intestinal submucosa at -80°C to -20°C for 2-24 hours, then allowing to thaw in normal saline at 20-30°C, and repeating the freeze-thaw cycle for 3-5 times; then mixing the small intestinal submucosa with a 0.05-10 mg/mL oxidase solution at 2-6°C for 20-30 hours; then taking the small intestinal submucosa out and mixing with a 0.05-5 mg/mL surfactant solution at 30-40°C for 1-5 hours, washing and drying; and
(4) placing the small intestinal submucosa after being treated in step (3) on a mold, and lyophilizing the mold containing the small intestinal submucosa in a lyophilizer; a procedure of the lyophilizing includes: pre-freezing to -45°C to -25°C, and holding for 1-2 hours; setting the pressure to 20-25 Pa, and adjusting the temperature to -25°C to -15°C and holding for 5-7 hours; and finally adjusting the temperature to 0°C, and holding for 4-6 hours.

In a second aspect, the present application provides a decellularized matrix material, and the decellularized matrix material is prepared by the preparation method for a decellularized matrix material described in the first aspect.

In a third aspect, the present application provides use of the decellularized matrix material described in the second aspect in the preparation of a medical implant material.

The decellularized matrix material prepared by the present application has a microscopically porous structure, provides a scaffold for cell growth, and is capable of being applied to any tissue repaired with an anastomat, such as lung tissues, gastric tissues, intestinal tissues, and skin tissues, to protect the wound, prevent the leakage, and accelerate the healing process. Additionally, the decellularized matrix material can also be applied to various tissue defects, including oral cavity, dura mater, pelvic cavity, ureter, bladder, inguinal hernia, uterus, breast and other tissues.

Compared with the prior art, the present application has the following beneficial effects:
in the present application, the physical swelling, physical freezing and thawing, and chemical elution are combined, and additionally, the oxidase is added at an appropriate time during the decellularization treatment process; the effective synergistic effect among various steps guarantees a high decellularization efficiency as well as an intact collagen structure in the extracellular matrix; moreover, the ZnCl₂ solution is creatively used in swelling to further improve the porosity and purification efficiency.

### BRIEF DESCRIPTION OF DRAWINGS

FIG. 1 shows the electron microscopy morphology of the decellularized matrix material prepared in Example 1;
FIG. 2 shows the electron microscopy morphology of the decellularized matrix material prepared in Example 2;
FIG. 3 shows the electron microscopy morphology of the decellularized matrix material prepared in Example 3;
FIG. 4 shows the electron microscopy morphology of the decellularized matrix material prepared in Comparative Example 1;
FIG. 5 shows the electron microscopy morphology of the decellularized matrix material prepared in Comparative Example 2;
FIG. 6 shows the electron microscopy morphology of the decellularized matrix material prepared in Comparative Example 3.

### DETAILED DESCRIPTION

In order to further elaborate the technical means adopted in the present application and their effects, the present application is further described hereinafter in terms of the examples and drawings. It is to be understood that the specific embodiments described herein are only used for explaining the present application, and are not a limitation to the present application.

The examples without specific techniques or conditions indicated are implemented in accordance with the techniques or conditions described in the literature in the art or in accordance with the product specification. The used reagents or instruments without manufacturer indicated are regular products commercially available through formal channels.

### Example 1

In this example, a decellularized matrix material is prepared, including the following steps:
(A1) Pre-treatment of matrix material
   a porcine small intestinal tissue, which was soft and smooth and did not have damage, ulcer or lesions, was used; the small intestinal wall tissue was washed with normal saline and drained, then the serosa, muscularis and mucosa of the tissue were removed off, and subsequently, the retained small intestinal submucosa was rinsed with normal saline until there was no visible unnecessary tissue or contamination, and drained;
(A2) Bacteria and viruses inactivation
   the small intestinal submucosa obtained from step (A1) was immersed in normal saline and irradiated with ultraviolet light for 15 minutes, wherein a volume ratio of the small intestinal submucosa to the normal saline was 1: 200; the small intestinal submucosa was taken out and immersed in an aqueous antibiotic solution containing 10 mg/L penicillin, 10 mg/L streptomycin, and 10 mg/L chloramphenicol at 25°C for 24 hours, wherein a volume ratio of the small intestinal submucosa to the aqueous antibiotic solution was 1: 150; the small intestinal submucosa was taken out and washed three times with sterile water for 10 minutes each time, and then the small intestinal submucosa was immersed in an aqueous solution of 5wt% peroxyacetic acid at 25°C for 2 hours, wherein a volume ratio of the small intestinal submucosa to the aqueous solution of peroxyacetic acid was 1: 120; the small intestinal submucosa was taken out and washed 3 times under ultrasonic conditions for 10 minutes each time, and drained;
(A3) Reparative decellularization
   the small intestinal submucosa treated in step (A2) was immersed in an aqueous ZnCl₂ solution and incubated in a shaker at 37°C for 2 hours (a concentration of the ZnCl₂ solution was 6 M), wherein a volume ratio of the small intestinal submucosa to the aqueous ZnCl₂ solution was 1: 200, and the small intestinal submucosa was taken out and washed 3 times with normal saline for 5 minutes each time;
   the above small intestinal submucosa was incubated at -80°C for 2 hours, and then placed in a sufficient amount of 25°C normal saline to thaw; such freeze-thaw cycle was repeated for 4 times;
   the small intestinal submucosa was taken out and immersed in an aqueous oxidase solution containing 1 mg/mL of D-amino acid oxidase and 1 mg/mL of L-amino acid oxidase at 4°C for 24 hours, wherein a volume ratio of the small intestinal submucosa to the aqueous oxidase solution was 1: 200;
   the small intestinal submucosa was taken out, immersed in an aqueous surfactant solution containing 0.5 mg/mL of Polysorbate 60 and 0.7 mg/mL of PEG400, and incubated in a shaker at 37°C for 2 hours, wherein a volume ratio of the small intestinal submucosa to the aqueous surfactant solution was 1: 200; and
   the small intestinal submucosa was taken out and washed 3 times under ultrasonic conditions for 10 minutes each time, and then drained;
(A4) Fixation molding
   two layers of the small intestinal submucosa obtained from step (A3) were placed on a mold;
(A5) Vacuum lyophilization
   the mold containing the small intestinal submucosa in step (A4) was placed in a lyophilizer, pre-freezed to -25°C and held for 2 hours first; then the pressure inside the chamber was adjusted to 25 Pa by turning on a vacuum pump, the temperature was adjusted to -15°C and held for 7 hours, and finally, the temperature was adjusted to 0°C and held for 4 hours; then the processing was completed and the decellularized matrix material was obtained.

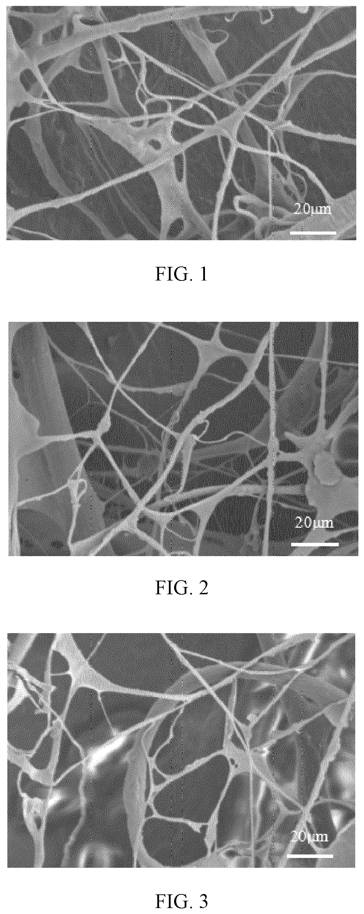

The decellularized matrix material was subjected to scanning electron microscopy (SEM) detection. The scanning electron microscope was Zeiss Sigma 300 ASAP; the scanning parameters were: a voltage of 3.00 kV, a working distance of 14.5 mm, and a magnification of 8000 times. The scanning area was the surface morphology of the patch, as shown in FIG. 1.

### Example 2

In this example, a decellularized matrix material is prepared, including the following steps:
(A1) Pre-treatment of matrix material
   a porcine small intestinal tissue, which was soft and smooth and did not have damage, ulcer, or lesions, was used; the small intestinal wall tissue was washed with normal saline, and drained, then the serosa, muscularis and mucosa of the tissue were removed off, and subsequently, the retained small intestinal submucosa was rinsed with normal saline until there was no visible unnecessary tissue or contamination, and drained;
(A2) Bacteria and viruses inactivation
   the small intestinal submucosa obtained from step (A1) was immersed in normal saline and irradiated with ultraviolet light for 20 minutes, wherein a volume ratio of the small intestinal submucosa to the normal saline was 1: 160; the small intestinal submucosa was taken out and immersed in an aqueous antibiotic solution containing 15 mg/L penicillin and 10 mg/L vancomycin at 35°C for 12 hours, wherein a volume ratio of the small intestinal submucosa to the aqueous antibiotic solution was 1: 150; the small intestinal submucosa was taken out and washed three times with sterile water for 10 minutes each time, and then the small intestinal submucosa was immersed in an aqueous solution of 5wt% peroxyacetic acid at 25°C for 2 hours, wherein a volume ratio of the small intestinal submucosa to the aqueous solution of peroxyacetic acid was 1: 150; the small intestinal submucosa was taken out and washed 3 times under ultrasonic conditions for 10 minutes each time, and then drained;
(A3) Reparative decellularization
   the small intestinal submucosa treated in step (A2) was immersed in an aqueous ZnCl₂ solution and incubated in a shaker at 20°C for 5 hours (a concentration of the ZnCl₂ solution was 4.5 M), wherein a volume ratio of the small intestinal submucosa to the aqueous ZnCl₂ solution was 1: 180, and the small intestinal submucosa was taken out and washed 3 times with normal saline for 5 minutes each time;
   the above small intestinal submucosa was incubated at -40°C for 7 hours, and then placed in a sufficient amount of 25°C normal saline to thaw; such freeze-thaw cycle was repeated for 3 times;
   the small intestinal submucosa was taken out and immersed in an aqueous oxidase solution containing 0.05 mg/mL of L-α-hydroxy acid oxidase at 4°C for 24 hours, wherein a volume ratio of the small intestinal submucosa to the aqueous oxidase solution was 1: 160;
   the small intestinal submucosa was taken out, immersed in an aqueous surfactant solution containing 0.05 mg/mL of Polysorbate 80, and incubated in a shaker at 37°C for 2 hours, wherein a volume ratio of the small intestinal submucosa to the aqueous surfactant solution was 1: 120; and
   the small intestinal submucosa was taken out and washed 3 times under ultrasonic conditions for 10 minutes each time, and then drained;
(A4) Fixation molding
   one layer of the small intestinal submucosa obtained from step (A3) was placed on a mold;
(A5) Vacuum lyophilization
   the mold containing the small intestinal submucosa in step (A4) was placed in a lyophilizer, pre-freezed to -35°C and held for 2 hours first; then the pressure inside the chamber was adjusted to 20 Pa by turning on a vacuum pump, the temperature was adjusted to -20°C and held for 5 hours, and finally, the temperature was adjusted to 0°C and held for 6 hours; then the processing was completed and the decellularized matrix material was obtained; the electron microscopy morphology is shown in FIG. 2.

### Example 3

In this example, a decellularized matrix material is prepared, including the following steps:
(A1) Pre-treatment of matrix material
   a porcine small intestinal tissue, which was soft and smooth and did not have damage, ulcer, or lesions, was used; the small intestinal wall tissue was washed with normal saline, and drained, then the serosa, muscularis, and mucosa of the tissue were removed off, and subsequently, the retained small intestinal submucosa was rinsed with normal saline until there was no visible unnecessary tissue or contamination, and drained;
(A2) Bacteria and viruses inactivation
   the small intestinal submucosa obtained from step (A1) was immersed in normal saline and irradiated with ultraviolet light for 20 minutes, wherein a volume ratio of the small intestinal submucosa to the normal saline was 1: 130; the small intestinal submucosa was taken out and immersed in an aqueous antibiotic solution containing 8 mg/L streptomycin, 6 mg/L vancomycin, and 12 mg/L chloramphenicol at 25°C for 20 hours, wherein a volume ratio of the small intestinal submucosa to the aqueous antibiotic solution was 1: 200; the small intestinal submucosa was taken out and washed three times with sterile water for 10 minutes each time, and then the small intestinal submucosa was immersed in an aqueous solution of 5wt% peroxyacetic acid at 25°C for 2 hours, wherein a volume ratio of the small intestinal submucosa to the aqueous solution of peroxyacetic acid was 1: 140; the small intestinal submucosa was taken out and washed 3 times under ultrasonic conditions for 10 minutes each time, and then drained;
(A3) Reparative decellularization
   the small intestinal submucosa treated in step (A2) was immersed in an aqueous ZnCl₂ solution and incubated in a shaker at 40°C for 2 hours (a concentration of the ZnCl₂ solution was 3 M), wherein a volume ratio of the small intestinal submucosa to the aqueous ZnCl₂ solution was 1: 150, and the small intestinal submucosa was taken out and washed 3 times with normal saline for 5 minutes each time;
   the above small intestinal submucosa was incubated at -20°C for 24 hours, and then placed in a sufficient amount of 25°C normal saline to thaw; such freeze-thaw cycle was repeated for 4 times;
   the small intestinal submucosa was taken out and immersed in an aqueous oxidase solution containing 7.5 mg/mL of urate oxidase and 2.5 mg/mL D-amino acid oxidase at 4°C for 24 hours, wherein a volume ratio of the small intestinal submucosa to the aqueous oxidase solution was 1: 100;
   the small intestinal submucosa was taken out, immersed in an aqueous surfactant solution containing 2.5 mg/mL of Polysorbate 80 and 2.5 mg/mL of PEG400, and incubated in a shaker at 37°C for 2 hours, wherein a volume ratio of the small intestinal submucosa to the aqueous surfactant solution was 1: 160; and
   the small intestinal submucosa was taken out and washed 3 times under ultrasonic conditions for 10 minutes each time, and then drained;
(A4) Fixation molding
   two layers of the small intestinal submucosa obtained from step (A3) were placed on a mold;
(A5) Vacuum lyophilization
   the mold containing the small intestinal submucosa in step (A4) was placed in a lyophilizer, pre-freezed to -45°C and held for 1 hour first; then the pressure inside the chamber was adjusted to 25 Pa by turning on a vacuum pump, the temperature was adjusted to -20°C and held for 7 hours, and finally, the temperature was adjusted to 0°C and held for 5 hours; then the processing was completed and the decellularized matrix material was obtained; the electron microscopy morphology is shown in FIG. 3.

### Example 4

Compared with Example 2, the difference only lies in that for the swelling operation of (A3) reparative decellularization, sodium chloride was used to replace zinc chloride; other operations are the same as in Example 2;
(A3) Reparative decellularization
   the small intestinal submucosa treated in step (A2) of Example 2 was immersed in an aqueous NaCl solution and incubated in a shaker at 37°C for 2 hours (a concentration of the NaCl solution was 4.5 M), wherein a volume ratio of the small intestinal submucosa to the aqueous NaCl solution was 1: 180, and the small intestinal submucosa was taken out and washed 3 times with normal saline for 5 minutes each time;
   the above small intestinal submucosa was incubated at -40°C for 7 hours, and then placed in a sufficient amount of 25°C normal saline to thaw; such freeze-thaw cycle was repeated for 3 times;
   the small intestinal submucosa was taken out and immersed in an aqueous oxidase solution containing 2.5 mg/mL of L-α-hydroxy acid oxidase at 4°C for 24 hours, wherein a volume ratio of the small intestinal submucosa to the aqueous oxidase solution was 1: 160;
   the small intestinal submucosa was taken out, immersed in an aqueous surfactant solution containing 1.8 mg/mL of Polysorbate 80, and incubated in a shaker at 37°C for 2 hours, wherein a volume ratio of the small intestinal submucosa to the aqueous surfactant solution was 1: 120; and
   the small intestinal submucosa was taken out and washed 3 times under ultrasonic conditions for 10 minutes each time, and then drained;
(A4) Fixation molding
   one layer of the small intestinal submucosa obtained from step (A3) was placed on a mold;
(A5) Vacuum lyophilization
   the mold containing the small intestinal submucosa in step (A4) was placed in a lyophilizer, pre-freezed to -35°C and held for 2 hours first; then the pressure inside the chamber was adjusted to 20 Pa by turning on a vacuum pump, the temperature was adjusted to -20°C and held for 5 hours, and finally, the temperature was adjusted to 0°C and held for 6 hours; then the processing was completed and the decellularized matrix material was obtained.

### Example 5

Compared with Example 2, the difference only lies in that for the swelling operation of (A3) reparative decellularization, calcium chloride was used to replace zinc chloride; other operations are the same as in Example 2;
(A3) Reparative decellularization
   the small intestinal submucosa treated in step (A2) of Example 2 was immersed in an aqueous CaCl₂ solution and incubated in a shaker at 37°C for 2 hours (a concentration of the CaCl₂ solution was 4.5 M), wherein a volume ratio of the small intestinal submucosa to the aqueous CaCl₂ solution was 1: 180, and the small intestinal submucosa was taken out and washed 3 times with normal saline for 5 minutes each time;
   the above small intestinal submucosa was incubated at -40°C for 7 hours, and then placed in a sufficient amount of 25°C normal saline to thaw; such freeze-thaw cycle was repeated for 3 times;
   the small intestinal submucosa was taken out and immersed in an aqueous oxidase solution containing 2.5 mg/mL of L-α-hydroxy acid oxidase at 4°C for 24 hours, wherein a volume ratio of the small intestinal submucosa to the aqueous oxidase solution was 1: 160;
   the small intestinal submucosa was taken out, immersed in an aqueous surfactant solution containing 1.8 mg/mL of Polysorbate 80, and incubated in a shaker at 37°C for 2 hours, wherein a volume ratio of the small intestinal submucosa to the aqueous surfactant solution was 1: 120; and
   the small intestinal submucosa was taken out and washed 3 times under ultrasonic conditions for 10 minutes each time, and then drained;
(A4) Fixation molding
   one layer of the small intestinal submucosa obtained from step (A3) was placed on a mold;
(A5) Vacuum lyophilization
   the mold containing the small intestinal submucosa in step (A4) was placed in a lyophilizer, pre-freezed to -35°C and held for 2 hours first; then the pressure inside the chamber was adjusted to 20 Pa by turning on a vacuum pump, the temperature was adjusted to -20°C and held for 5 hours, and finally, the temperature was adjusted to 0°C and held for 6 hours; then the processing was completed and the decellularized matrix material was obtained.

### Example 6

Compared with Example 2, the difference only lies in that for the swelling operation of (A3) reparative decellularization, potassium chloride was used to replace zinc chloride; other operations are the same as in Example 2;
(A3) Reparative decellularization
   the small intestinal submucosa treated in step (A2) of Example 2 was immersed in an aqueous KCl solution and incubated in a shaker at 37°C for 2 hours (a concentration of the KCl solution was 4.5 M), wherein a volume ratio of the small intestinal submucosa to the aqueous KCl solution was 1: 180, and the small intestinal submucosa was taken out and washed 3 times with normal saline for 5 minutes each time;
   the above small intestinal submucosa was incubated at -40°C for 7 hours, and then placed in a sufficient amount of 25°C normal saline to thaw; such freeze-thaw cycle was repeated for 3 times;
   the small intestinal submucosa was taken out and immersed in an aqueous oxidase solution containing 2.5 mg/mL of L-α-hydroxy acid oxidase at 4°C for 24 hours, wherein a volume ratio of the small intestinal submucosa to the aqueous oxidase solution was 1: 160;
   the small intestinal submucosa was taken out, immersed in an aqueous surfactant solution containing 1.8 mg/mL of Polysorbate 80, and incubated in a shaker at 37°C for 2 hours, wherein a volume ratio of the small intestinal submucosa to the aqueous surfactant solution was 1: 120; and
   the small intestinal submucosa was taken out and washed 3 times under ultrasonic conditions for 10 minutes each time, and then drained;
(A4) Fixation molding
   one layer of the small intestinal submucosa obtained from step (A3) was placed on a mold;
(A5) Vacuum lyophilization
   the mold containing the small intestinal submucosa in step (A4) was placed in a lyophilizer, pre-freezed to -35°C and held for 2 hours first; then the pressure inside the chamber was adjusted to 20 Pa by turning on a vacuum pump, the temperature was adjusted to -20°C and held for 5 hours, and finally, the temperature was adjusted to 0°C and held for 6 hours; then the processing was completed and the decellularized matrix material was obtained.

### Example 7

Compared with Example 2, the difference only lies in that for the swelling operation of (A3) reparative decellularization, magnesium chloride was used to replace zinc chloride; other operations are the same as in Example 2;
(A3) Reparative decellularization
   the small intestinal submucosa treated in step (A2) of Example 2 was immersed in an aqueous MgCl₂ solution and incubated in a shaker at 37°C for 2 hours (a concentration of the MgCl₂ solution was 4.5 M), wherein a volume ratio of the small intestinal submucosa to the aqueous MgCl₂ solution was 1: 180, and the small intestinal submucosa was taken out and washed 3 times with normal saline for 5 minutes each time;
   the above small intestinal submucosa was incubated at -40°C for 7 hours, and then placed in a sufficient amount of 25°C normal saline to thaw; such freeze-thaw cycle was repeated for 3 times;
   the small intestinal submucosa was taken out and immersed in an aqueous oxidase solution containing 2.5 mg/mL of L-α-hydroxy acid oxidase at 4°C for 24 hours, wherein a volume ratio of the small intestinal submucosa to the aqueous oxidase solution was 1: 160;
   the small intestinal submucosa was taken out, immersed in an aqueous surfactant solution containing 1.8 mg/mL of Polysorbate 80, and incubated in a shaker at 37°C for 2 hours, wherein a volume ratio of the small intestinal submucosa to the aqueous surfactant solution was 1: 120; and
   the small intestinal submucosa was taken out and washed 3 times under ultrasonic conditions for 10 minutes each time, and then drained;
(A4) Fixation molding
   one layer of the small intestinal submucosa obtained from step (A3) was placed on a mold;
(A5) Vacuum lyophilization
   the mold containing the small intestinal submucosa in step (A4) was placed in a lyophilizer, pre-freezed to -35°C and held for 2 hours first; then the pressure inside the chamber was adjusted to 20 Pa by turning on a vacuum pump, the temperature was adjusted to -20°C and held for 5 hours, and finally, the temperature was adjusted to 0°C and held for 6 hours; then the processing was completed and the decellularized matrix material was obtained.

### Comparative Example 1

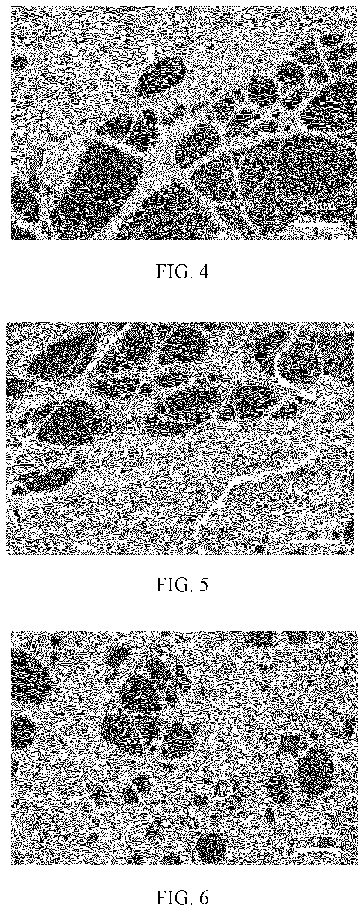

Compared with Example 1, the difference only lies in that in (A3) reparative decellularization, the surfactant treatment was performed first, and then the oxidase treatment was performed; other operations are the same as in Example 1;
(A3) Reparative decellularization
   the small intestinal submucosa treated in step (A2) of Example 1 was immersed in an aqueous ZnCl₂ solution and incubated in a shaker at 37°C for 2 hours (a concentration of the ZnCl₂ solution was 6 M), wherein a volume ratio of the small intestinal submucosa to the aqueous ZnCl₂ solution was 1: 200, and the small intestinal submucosa was taken out and washed 3 times with normal saline for 5 minutes each time;
   the above small intestinal submucosa was incubated at -80°C for 2 hours, and then placed in a sufficient amount of 25°C normal saline to thaw; such freeze-thaw cycle was repeated for 4 times;
   the small intestinal submucosa was taken out, immersed in an aqueous surfactant solution containing 0.5 mg/mL of Polysorbate 60 and 0.7 mg/mL of PEG400, and incubated in a shaker at 37°C for 2 hours, wherein a volume ratio of the small intestinal submucosa to the aqueous
   surfactant solution was 1: 200;
   the small intestinal submucosa was taken out and immersed in an aqueous oxidase solution containing 1 mg/mL of D-amino acid oxidase and 1 mg/mL of L-amino acid oxidase at 4°C for 24 hours, wherein a volume ratio of the small intestinal submucosa to the aqueous oxidase solution was 1: 200; and
   the small intestinal submucosa was taken out and washed 3 times under ultrasonic conditions for 10 minutes each time, and then drained;
(A4) Fixation molding
   two layers of the small intestinal submucosa obtained from step (A3) were placed on a mold;
(A5) Vacuum lyophilization
   the mold containing the small intestinal submucosa in step (A4) was placed in a lyophilizer, pre-freezed to -25°C and held for 2 hours first; then the pressure inside the chamber was adjusted to 25 Pa by turning on a vacuum pump, the temperature was adjusted to -15°C and held for 7 hours, and finally, the temperature was adjusted to 0°C and held for 4 hours; then the processing was completed and the decellularized matrix material was obtained; the electron microscopy morphology is shown in FIG. 4.

### Comparative Example 2

Compared with Example 1, the difference only lies in that in (A3) reparative decellularization, the oxidase treatment was performed before the swelling operation; other operations are the same as in Example 1;
(A3) Reparative decellularization
   the small intestinal submucosa treated in step (A2) of Example 1 was immersed in an aqueous oxidase solution containing 1 mg/mL of D-amino acid oxidase and 1 mg/mL of L-amino acid oxidase at 4°C for 24 hours, wherein a volume ratio of the small intestinal submucosa to the aqueous oxidase solution was 1: 200;
   the small intestinal submucosa was taken out and immersed in an aqueous ZnCl₂ solution and incubated in a shaker at 37°C for 2 hours (a concentration of the ZnCl₂ solution was 6 M), wherein a volume ratio of the small intestinal submucosa to the aqueous ZnCl₂ solution was 1: 200, and the small intestinal submucosa was taken out and washed 3 times with normal saline for 5 minutes each time;
   the above small intestinal submucosa was incubated at -80°C for 2 hours, and then placed in a sufficient amount of 25°C normal saline to thaw; such freeze-thaw cycle was repeated for 4 times;
   the small intestinal submucosa was taken out, immersed in an aqueous surfactant solution containing 0.5 mg/mL of Polysorbate 60 and 0.7 mg/mL of PEG400, and incubated in a shaker at 37°C for 2 hours, wherein a volume ratio of the small intestinal submucosa to the aqueous surfactant solution was 1: 200; and
   the small intestinal submucosa was taken out and washed 3 times under ultrasonic conditions for 10 minutes each time, and then drained;
(A4) Fixation molding
   two layers of the small intestinal submucosa obtained from step (A3) were placed on a mold;
(A5) Vacuum lyophilization
   the mold containing the small intestinal submucosa in step (A4) was placed in a lyophilizer, pre-freezed to -25°C and held for 2 hours first; then the pressure inside the chamber was adjusted to 25 Pa by turning on a vacuum pump, the temperature was adjusted to -15°C and held for 7 hours, and finally, the temperature was adjusted to 0°C and held for 4 hours; then the processing was completed and the decellularized matrix material was obtained; the electron microscopy morphology is shown in FIG. 5.

### Comparative Example 3

Compared with Example 1, the difference only lies in that in (A3) reparative decellularization, the oxidase treatment was not performed; other operations are the same as in Example 1;
(A3) Reparative decellularization
   the small intestinal submucosa treated in step (A2) of Example 1 was immersed in an aqueous ZnCl₂ solution and incubated in a shaker at 37°C for 2 hours (a concentration of the ZnCl₂ solution was 6 M), wherein a volume ratio of the small intestinal submucosa to the aqueous ZnCl₂ solution was 1: 200, and the small intestinal submucosa was taken out and washed 3 times with normal saline for 5 minutes each time;
   the above small intestinal submucosa was incubated at -80°C for 2 hours, and then placed in a sufficient amount of 25°C normal saline to thaw; such freeze-thaw cycle was repeated for 4 times;
   the small intestinal submucosa was taken out, immersed in an aqueous surfactant solution containing 0.5 mg/mL of Polysorbate 60 and 0.7 mg/mL of PEG400, and incubated in a shaker at 37°C for 2 hours, wherein a volume ratio of the small intestinal submucosa to the aqueous surfactant solution was 1: 200; and
   the small intestinal submucosa was taken out and washed 3 times under ultrasonic conditions for 10 minutes each time, and then drained;
(A4) Fixation molding
   two layers of the small intestinal submucosa obtained from step (A3) were placed on a mold;
(A5) Vacuum lyophilization
   the mold containing the small intestinal submucosa in step (A4) was placed in a lyophilizer, pre-freezed to -25°C and held for 2 hours first; then the pressure inside the chamber was adjusted to 25 Pa by turning on a vacuum pump, the temperature was adjusted to -15°C and held for 7 hours, and finally, the temperature was adjusted to 0°C and held for 4 hours; then the processing was completed and the decellularized matrix material was obtained; the electron microscopy morphology is shown in FIG. 6.

### Test Example

In this test example, the decellularized matrix materials prepared by the above examples and comparative examples were tested. The porosity of the decellularized matrix materials was tested by a high-performance full automatic mercury intrusion porosimeter; the number of cells in the field of view was counted by an optical microscope at 100×visual field; the amount of residual DNA was detected by PCR; and the collagen structure was detected by a scanning electron microscopy. The results are shown in Table 1. It can be seen that by controlling the particular preparation process in the present application, and by strictly controlling and sequentially performing the swelling treatment, freeze-thawing treatment, oxidase immersion treatment, and surfactant treatment, the porosity of the decellularized matrix material is significantly improved, and the number of cells in visual field of view and the amount of residual DNA are lower, indicating that in the present application, the combination of the physical swelling, physical freezing and thawing, and chemical elution, and the addition of oxidase at an appropriate time during the decellularization treatment process, the effective synergistic effect among various steps guarantees a high decellularization efficiency as well as an intact collagen structure in the extracellular matrix. As can be seen from the examples and comparative examples, the collagen frameworks of the examples are more intact, while the collagen frameworks of the comparative examples partially collapses; an intact collagen framework is more conducive to cell growth and wound healing after the patch is implanted into the body. Moreover, the ZnCl₂ solution is creatively used in swelling to further improve the porosity and purification efficiency.

**Table 1**

| | Porosity | Number of cells per field of view | Residual DNA |
|---|---|---|---|
| Example 1 | 94% | 0 | 2 ng/mg |
| Example 2 | 95% | 0 | 2 ng/mg |
| Example 3 | 92% | 1 | 3 ng/mg |
| Example 4 | 85% | 1 | 2 ng/mg |
| Example 5 | 85% | 2 | 3 ng/mg |
| Example 6 | 83% | 1 | 3 ng/mg |
| Example 7 | 83% | 2 | 1 ng/mg |
| Comparative Example 1 | 82% | 1 | 2 ng/mg |
| Comparative Example 2 | 83% | 2 | 1 ng/mg |
| Comparative Example 3 | 80% | 1 | 3 ng/mg |

In summary, in the present application, the combination of physical swelling, physical freezing and thawing, and chemical elution guarantees a high decellularization efficiency; moreover, by adding the oxidase at an appropriate time during the decellularization treatment process, the oxidase thoroughly permeates into the decellularized matrix, and thereby plays a buffering role in the tissue when the surfactant is applied, ensuring the intactness of the collagen structure in the extracellular matrix.

## Claims

1. A preparation method for a decellularized matrix material, comprising:
subjecting small intestinal submucosa to pre-treatment, and subjecting the small intestinal submucosa after the pre-treatment to swelling treatment, freeze-thaw treatment, oxidase immersion treatment, and surfactant treatment in sequence, so as to obtain the decellularized matrix material, wherein the swelling treatment comprises: mixing and incubating the small intestinal submucosa with a hypertonic solution, wherein the hypertonic solution comprises any one or a combination of at least two of a ZnCl₂ solution, a NaCl solution, a CaCl₂ solution, a KCl solution, or a MgCl₂ solution and the hypertonic solution has a concentration of 3-6 M.

2. The preparation method for a decellularized matrix material according to claim 1, wherein the pre-treatment comprises washing and drying;
wherein the pre-treatment further comprises bacteria and viruses inactivation;
the bacteria and viruses inactivation comprises: irradiating the small intestinal submucosa with ultraviolet light, then incubating with an antibiotic solution, washing, and incubating with peroxyacetic acid;
the antibiotic solution contains any one or a combination of at least two of gentamicin, vancomycin, penicillin, streptomycin, cefoxitin, or chloramphenicol; and
the antibiotic solution has a total concentration of 1-100 mg/L.

3. The preparation method for a decellularized matrix material according to claim 1, wherein the incubation is performed for a period of 2-5 hours at a temperature of 20-40°C.

4. The preparation method for a decellularized matrix material according to claim 1, wherein the freeze-thaw treatment comprises: incubating the small intestinal submucosa at -80°C to -20°C for 2-24 hours, then allowing the small intestinal submucosa to thaw in normal saline at 20-30°C, and repeating the freeze-thaw cycle for 3-5 times.

5. The preparation method for decellularized matrix material according to claim 1, wherein the oxidase comprises any one or a combination of at least two of lipoxygenase, urate oxidase, D-amino acid oxidase, L-amino acid oxidase, or L-α-hydroxy acid oxidase;
wherein the oxidase has a working concentration of 0.05-10 mg/mL.

6. The preparation method for a decellularized matrix material according to claim 1, wherein the surfactant comprises any one or a combination of at least two of a saponifiable matter, polyethylene glycol, or polysorbate;
wherein the surfactant has a working concentration of 0.05-5 mg/mL.

7. The preparation method for a decellularized matrix material according to claim 1, wherein after the surfactant treatment, the preparation method further comprises steps of fixation molding and vacuum lyophilizing;
wherein the fixation molding comprises: placing at least one layer of small intestinal submucosa on a mold;
the vacuum lyophilizing comprises: lyophilizing the mold containing the small intestinal submucosa in a lyophilizer; and
a procedure of the lyophilizing comprises: pre-freezing to -45°C to -25°C, and holding for 1-2 hours; setting a pressure to 20-25 Pa, and adjusting a temperature to -25°C to -15°C and holding for 5-7 hours; and finally adjusting a temperature to 0°C, and holding for 4-6 hours.

8. The preparation method for a decellularized matrix material according to any one of claims 1-7, wherein the preparation method comprises the following steps:
(1) washing and drying the small intestinal submucosa;
(2) irradiating the small intestinal submucosa after being treated in step (1) with ultraviolet light, then incubating with an antibiotic solution, washing, incubating with peroxyacetic acid, and washing and drying;
(3) mixing the small intestinal submucosa after being treated in step (2) with a 3-6 M ZnCl₂ solution and incubating at 20-30°C for 2-5 hours, and washing; subsequently incubating the small intestinal submucosa at -80°C to -20°C for 2-24 hours, then allowing to thaw in normal saline at 20-30°C, and repeating the freeze-thaw cycle for 3-5 times; then mixing the small intestinal submucosa with a 0.05-10 mg/mL oxidase solution at 2-6°C for 20-30 hours; then taking the small intestinal submucosa out and mixing with a 0.05-5 mg/mL surfactant solution at 30-40°C for 1-5 hours, washing and drying; and
(4) placing the small intestinal submucosa after being treated in step (3) on a mold, and lyophilizing the mold containing the small intestinal submucosa in a lyophilizer; a procedure of the lyophilizing comprises: pre-freezing to -45°C to -25°C, and holding for 1-2 hours; setting the pressure to 20-25 Pa, and adjusting the temperature to -25°C to -15°C and holding for 5-7 hours; and finally adjusting the temperature to 0°C, and holding for 4-6 hours.

9. A decellularized matrix material, which is prepared by the preparation method for a decellularized matrix material according to any one of claims 1-8.

10. The decellularized matrix material according to claim 9 for use in the preparation of a medical implant material.

## Patentansprüche

1. Herstellungsverfahren für ein dezellularisiertes Matrixmaterial, umfassend:
Unterziehen von Dünndarmsubmukosa einer Vorbehandlung und Unterziehen der Dünndarmsubmukosa nach der Vorbehandlung nacheinander einer Quellbehandlung, einer Gefrier-Auftau-Behandlung, einer Oxidase-Immersionsbehandlung und einer Tensidbehandlung, um das dezellularisierte Matrixmaterial zu erhalten, wobei die Quellbehandlung umfasst: Mischen und Inkubieren der Dünndarmsubmukosa mit einer hypertonischen Lösung, wobei die hypertonische Lösung eine beliebige einzelne oder eine Kombination von mindestens zwei von einer ZnCl₂-Lösung, einer NaCl-Lösung, einer CaCl₂-Lösung, einer KCl-Lösung oder einer MgCl₂-Lösung umfasst und die hypertonische Lösung eine Konzentration von 3-6 M aufweist.

2. Herstellungsverfahren für ein dezellularisiertes Matrixmaterial nach Anspruch 1, wobei die Vorbehandlung Waschen und Trocknen umfasst;
wobei die Vorbehandlung ferner eine Inaktivierung von Bakterien und Viren umfasst; die Inaktivierung von Bakterien und Viren umfasst: Bestrahlen der Dünndarmsubmukosa mit ultraviolettem Licht, anschließendes Inkubieren mit einer Antibiotikalösung, Waschen und Inkubieren mit Peroxyessigsäure;
die Antibiotikalösung enthält eine beliebige einzelne oder eine Kombination von mindestens zwei von Gentamicin, Vancomycin, Penicillin, Streptomycin, Cefoxitin oder Chloramphenicol; und
die Antibiotikalösung weist eine Gesamtkonzentration von 1-100 mg/L auf.

3. Herstellungsverfahren für ein dezellularisiertes Matrixmaterial nach Anspruch 1, wobei das Inkubieren für einen Zeitraum von 2-5 Stunden bei einer Temperatur von 20-40°C durchgeführt wird.

4. Herstellungsverfahren für ein dezellularisiertes Matrixmaterial nach Anspruch 1, wobei die Gefrier-Auftau-Behandlung umfasst: Inkubieren der Dünndarmsubmukosa bei -80°C bis - 20°C für 2-24 Stunden, anschließendes Auftauen der Dünndarmsubmukosa in physiologischer Kochsalzlösung bei 20-30°C und Wiederholen des Gefrier-Auftau-Zyklus 3-5-mal.

5. Herstellungsverfahren für ein dezellularisiertes Matrixmaterial nach Anspruch 1, wobei die Oxidase eine beliebige einzelne oder eine Kombination von mindestens zwei von Lipoxygenase, Uratoxidase, D-Aminosäureoxidase, L-Aminosäureoxidase oder L-α-Hydroxysäureoxidase umfasst;
wobei die Oxidase eine Arbeitskonzentration von 0,05-10 mg/mL aufweist.

6. Herstellungsverfahren für ein dezellularisiertes Matrixmaterial nach Anspruch 1, wobei das Tensid eine beliebige einzelne oder eine Kombination von mindestens zwei von einer verseifbaren Substanz, Polyethylenglykol oder Polysorbat umfasst;
wobei das Tensid eine Arbeitskonzentration von 0,05-5 mg/mL aufweist.

7. Herstellungsverfahren für ein dezellularisiertes Matrixmaterial nach Anspruch 1, wobei das Herstellungsverfahren nach der Tensidbehandlung ferner Schritte des Fixierungsformens und des Vakuumgefriertrocknens umfasst;
wobei das Fixierungsformen umfasst: Platzieren von mindestens einer Lage Dünndarmsubmukosa auf einer Form;
das Vakuumgefriertrocknen umfasst: Gefriertrocknen der die Dünndarmsubmukosa enthaltenden Form in einem Gefriertrockner; und
ein Verfahren des Gefriertrocknens umfasst: Vorfrieren auf -45°C bis -25°C und Halten für 1-2 Stunden; Einstellen eines Drucks auf 20-25 Pa und Einstellen einer Temperatur auf -25°C bis -15°C und Halten für 5-7 Stunden; und schließlich Einstellen einer Temperatur auf 0°C und Halten für 4-6 Stunden.

8. Herstellungsverfahren für ein dezellularisiertes Matrixmaterial nach einem der Ansprüche 1 bis 7, wobei das Herstellungsverfahren die folgenden Schritte umfasst:
(1) Waschen und Trocknen der Dünndarmsubmukosa;
(2) Bestrahlen der in Schritt (1) behandelten Dünndarmsubmukosa mit ultraviolettem Licht, anschließendes Inkubieren mit einer Antibiotikalösung, Waschen, Inkubieren mit Peroxyessigsäure sowie Waschen und Trocknen;
(3) Mischen der in Schritt (2) behandelten Dünndarmsubmukosa mit einer 3-6 M ZnCl₂-Lösung und Inkubieren bei 20-30°C für 2-5 Stunden sowie Waschen; anschließendes Inkubieren der Dünndarmsubmukosa bei -80°C bis -20°C für 2-24 Stunden, anschließendes Auftauen in physiologischer Kochsalzlösung bei 20-30°C und Wiederholen des Gefrier-Auftau-Zyklus 3-5-mal; anschließend Mischen der Dünndarmsubmukosa mit einer 0,05-10 mg/mL Oxidaselösung bei 2-6°C für 20-30 Stunden; anschließendes Herausnehmen der Dünndarmsubmukosa und Mischen mit einer 0,05-5 mg/mL Tensidlösung bei 30-40°C für 1-5 Stunden sowie Waschen und Trocknen; und
(4) Platzieren der in Schritt (3) behandelten Dünndarmsubmukosa auf einer Form und Gefriertrocknen der die Dünndarmsubmukosa enthaltenden Form in einem Gefriertrockner; ein Verfahren des Gefriertrocknens umfasst: Vorfrieren auf -45°C bis - 25°C und Halten für 1-2 Stunden; Einstellen des Drucks auf 20 -25 Pa und Einstellen der Temperatur auf -25°C bis -15°C und Halten für 5-7 Stunden; und schließlich Einstellen der Temperatur auf 0°C und Halten für 4-6 Stunden.

9. Dezellularisiertes Matrixmaterial, hergestellt durch das Herstellungsverfahren für ein dezellularisiertes Matrixmaterial nach einem der Ansprüche 1 bis 8.

10. Dezellularisiertes Matrixmaterial nach Anspruch 9 zur Verwendung bei der Herstellung eines medizinischen Implantatmaterials.

## Revendications

1. Procédé de préparation d'un matériau de matrice décellularisée, comprenant :
soumission de la sous-muqueuse intestinale à un prétraitement, et soumission de la sous-muqueuse de l'intestin grêle après le prétraitement en séquence à un traitement par gonflement, à un traitement par congélation-décongélation, à un traitement par immersion à l'oxydase et à un traitement par tensioactif, de façon à obtenir le matériau de matrice décellularisée, dans lequel le traitement par gonflement comprend : mélange et incubation de la sous-muqueuse de l'intestin grêle avec une solution hypertonique, dans lequel la solution hypertonique comprend l'un quelconque ou une combinaison d'au moins deux éléments parmi une solution de ZnCl₂, une solution de NaCl, une solution de CaCl₂, une solution de KCl, ou une solution de MgCl₂ et la solution hypertonique présente une concentration de 3-6 M.

2. Procédé de préparation d'un matériau de matrice décellularisée selon la revendication 1, dans lequel le prétraitement comprend le lavage et le séchage ;
dans lequel le prétraitement comprend en outre l'inactivation de bactéries et de virus ; l'inactivation de bactéries et de virus comprend : irradiation de la sous-muqueuse de l'intestin grêle avec une lumière ultraviolette, puis incubation avec une solution antibiotique, lavage et incubation avec de l'acide peroxyacétique ;
la solution antibiotique contient l'un quelconque ou une combinaison d'au moins deux éléments parmi la gentamicine, la vancomycine, la pénicilline, la streptomycine, la céfoxitine, ou le chloramphénicol ; et
la solution antibiotique présente une concentration totale de 1-100 mg/L.

3. Procédé de préparation d'un matériau de matrice décellularisée selon la revendication 1, dans lequel l'incubation est effectuée pendant une période de 2-5 heures à une température de 20-40°C.

4. Procédé de préparation d'un matériau de matrice décellularisée selon la revendication 1, dans lequel le traitement par congélation-décongélation comprend : incubation de la sous-muqueuse de l'intestin grêle à -80°C à -20°C pendant 2-24 heures, puis laisser la sous-muqueuse de l'intestin grêle se décongeler dans une solution saline normale à 20-30°C, et répétition du cycle congélation-décongélation 3-5 fois.

5. Procédé de préparation d'un matériau de matrice décellularisée selon la revendication 1, dans lequel l'oxydase comprend l'un quelconque ou une combinaison d'au moins deux éléments parmi la lipoxygénase, l'urate oxydase, l'oxydase d'acide D-aminé, l'oxydase d'acide L-aminé ou la L-α-hydroxyacide oxydase ;
dans laquelle l'oxydase présente une concentration de travail de 0,05-10 mg/mL.

6. Procédé de préparation d'un matériau de matrice décellularisée selon la revendication 1, dans lequel le tensioactif comprend l'un quelconque ou une combinaison d'au moins deux éléments parmi une matière saponifiable, un polyéthylène glycol ou un polysorbate ;
dans lequel le tensioactif présente une concentration de travail de 0,05-5 mg/mL.

7. Procédé de préparation d'un matériau de matrice décellularisée selon la revendication 1, dans lequel, après le traitement par tensioactif, le procédé de préparation comprend en outre des étapes de moulage de fixation et de lyophilisation sous vide ;
dans lequel le moulage de fixation comprend : placement d'au moins une couche de sous-muqueuse de l'intestin grêle sur un moule ;
la lyophilisation sous vide comprend : lyophilisation du moule contenant la sous-muqueuse de l'intestin grêle dans un lyophilisateur ; et
une procédure de lyophilisation comprend : pré-congélation à -45°C à -25°C, et maintien pendant 1-2 heures ; réglage d'une pression à 20-25 Pa, et régulation d'une température à -25°C à -15°C et maintien pendant 5-7 heures ; et enfin régulation d'une température à 0°C, et maintien pendant 4-6 heures.

8. Procédé de préparation d'un matériau de matrice décellularisée selon l'une quelconque des revendications 1-7, dans lequel le procédé de préparation comprend les étapes suivantes :
(1) lavage et séchage de la sous-muqueuse de l'intestin grêle ;
(2) irradiation de la sous-muqueuse de l'intestin grêle après son traitement à l'étape (1) avec une lumière ultraviolette, puis incubation avec une solution antibiotique, lavage, incubation avec de l'acide peroxyacétique, et lavage et séchage ;
(3) mélange de la sous-muqueuse de l'intestin grêle après son traitement à l'étape (2) avec une solution de ZnCl₂ à 3-6 M et incubation à 20-30°C pendant 2-5 heures, et lavage ; par la suite, incubation de la sous-muqueuse de l'intestin grêle à -80°C à -20°C pendant 2-24 heures, puis laisser se décongeler dans une solution saline normale à 20-30°C, et répétition du cycle congélation-décongélation 3-5 fois ; puis mélange de la sous-muqueuse de l'intestin grêle avec une solution d'oxydase de 0,05-10 mg/mL à 2-6°C pendant 20-30 heures ; puis prélèvement de la sous-muqueuse de l'intestin grêle et mélange avec une solution de tensioactif de 0,05-5 mg/mL à 30-40°C pendant 1-5 heures, lavage et séchage ; et
(4) placement de la sous-muqueuse de l'intestin grêle après son traitement à l'étape (3) sur un moule, et lyophilisation du moule contenant la sous-muqueuse de l'intestin grêle dans un lyophilisateur ; une procédure de lyophilisation comprend : pré-congélation à -45°C à - 25°C, et maintien pendant 1-2 heures ; réglage de la pression à 20-25 Pa, et régulation de la température à -25°C à -15°C et maintien pendant 5-7 heures ; et enfin régulation de la température à 0°C, et maintien pendant 4-6 heures.

9. Matériau de matrice décellularisée, qui est préparé par le procédé de préparation d'un matériau de matrice décellularisée selon l'une quelconque des revendications 1-8.

10. Matériau de matrice décellularisée selon la revendication 9, pour utilisation dans la préparation d'un matériau d'implant médical.
